Europäisches Patentamt

(19) **European Patent Office**

**Office européen des brevets**

(11) Numéro de publication : **0 140 727**
**B1**

(12) **FASCICULE DE BREVET EUROPÉEN**

(45) Date de publication du fascicule du brevet :
**11.11.87**

(51) Int. Cl.⁴ : **A 61 M   5/14**

(21) Numéro de dépôt : **84401671.7**

(22) Date de dépôt : **14.08.84**

(54) **Dispositif d'injection de fluide, apte à être implanté.**

(30) Priorité : **02.09.83 FR 8314080**

(43) Date de publication de la demande :
**08.05.85 Bulletin 85/19**

(45) Mention de la délivrance du brevet :
**11.11.87 Bulletin 87/46**

(84) Etats contractants désignés :
**AT BE CH DE GB IT LI LU NL SE**

(56) Documents cités :
**DE-A- 2 309 749**
**FR-A- 2 269 922**
**US-A- 3 527 220**
**TRANS. AM. SOC. ARTIF. INTERN. ORGANS, vol. 24, 1978, pages 229-231; P.R. PERKINS et al.: "Design and initial testing of a totally implantable transcutaneously controllable insulin delivery device"**

(73) Titulaire : **Buffet, Jacques**
**28 avenue Thiers**
**F-93340 Le Raincy (FR)**

(72) Inventeur : **Buffet, Jacques**
**28 avenue Thiers**
**F-93340 Le Raincy (FR)**

(74) Mandataire : **Derambure, Christian**
**Cabinet BUGNION ASSOCIES SARL 116, boulevard Haussmann**
**F-75008 Paris (FR)**

Jouve, 18, rue St-Denis, 75001 Paris, France

**0 140 727**

## Description

La présente invention concerne un appareil pour fluides à injecter, pouvant être implanté dans le corps humain ou dans le corps d'un animal.

On sait que pour le traitement de diverses maladies, il est nécessaire d'administrer des drogues de façon continue ou de façon fractionnée et répétée au cours de la journée.

Ainsi il est de plus en plus courant d'injecter des fluides à l'aide d'un cathéter intramusculaire, intravasculaire ou intrapéritonéal. Ces fluides sont par exemple des solutions d'insuline pour le traitement du diabète, des anticoagulants pour les porteurs de prothèses valvulaires, des neuroleptiques, des médicaments antiarythmiques, des produits anticancéreux et des médicaments pour la restauration de la fertilité féminine.

Les doses injectées peuvent être fractionnées ou continues. Dans ce dernier cas, le débit doit être préréglé.

On utilise actuellement plusieurs sortes de dispositifs.

Les dispositifs les plus anciens sont des pompes externes, portées en bandouillière et reliées à un cathéter traversant la peau. Elles sont commandées par un circuit électrique alimenté par une pile ou un accumulateur. Cependant, de tels dispositifs ont pour inconvénient que le fluide peut être contaminé par des germes provenant de l'extérieur. En outre, la température du fluide n'est pas égale à la température du corps du patient puisque la pompe et le réservoir de fluide sont externes.

Enfin, le dispositif externe procure un handicap au patient qui ne peut exécuter toutes les activités souhaitées telles que baignade, douche, etc...

On a donc conçu plus récemment des pompes implantables qui peuvent être rechargées de l'extérieur à l'aide d'une seringue.

Ces pompes sont soit mécaniques soit électriques.

Les pompes mécaniques de la technique antérieure, telle que celle qui est décrite dans FR-A-2 306 712, sont constituées d'un boîtier séparé en deux chambres par une barrière imperméable flexible. Entre le boîtier et la barrière se trouve en permanence un fluide qui est en équilibre liquide/vapeur. Le volume à l'intérieur de la barrière constitue le réservoir de fluide à injecter. Ainsi la pression de vapeur du fluide en équilibre de phases exerce une pression constante sur le volume de fluide à injecter et ce dernier est expulsé à un débit constant par un capillaire de sortie. Lorsque le réservoir de fluide à injecter est vide, on injecte le fluide à l'aide d'une seringue, ce qui procure l'expansion du réservoir de fluide et entraîne la condensation de la vapeur du fluide en équilibre de phases et le cycle d'injection peut recommencer.

Le fluide en équilibre de phases est un gaz qui se liquifie au voisinage de la température du corps (37 °C) et est par exemple du butane.

De telles pompes ont donné de bons résultats mais cependant leur principal inconvénient est de délivrer le fluide de traitement à un débit non réglable et pratiquement constant.

Le document Trans. Am. Soc. Artif. Inter. Organs, vol 24, 1978, pages 229-231 (P.R. Perkinns et Al.) décrit également la possibilité d'adjoindre à une telle pompe une valve commandée magnétiquement à travers la peau et qui permet de varier le débit entre deux valeurs extrêmes. Cependant, des variations plus fines et l'injection d'une dose instantanée ne sont pas possibles avec une telle pompe.

De telles pompes sont à ce titre inutilisables pour traiter complètement la plupart des affections.

Les pompes électriques comportent en général une alimentation électrique telle qu'une pile, un circuit électronique permettant de régler le débit du fluide à injecter dans le temps, un réservoir de fluide à injecter et une pompe.

Ces pompes électriques ont pour inconvénient de comporter outre le réservoir de l'organe de pompage, un circuit électronique et une source d'énergie de longue durée. Ces pompes sont chères et volumineuses.

On connaît également du US-A- 3 527 220, un dispositif d'injection comprenant une partie implantable constituée d'une vessie, d'une pompe interposée sur une conduite d'alimentation. La vessie est remplie de l'extérieur par une seringue. La pompe est activée de l'extérieur par la rotation d'un aimant entraîné par un moteur. La pompe comporte un disque magnétique qui coopère à travers la peau avec l'aimant. De plus, des moyens d'indications lumineuses permettent de connaître la dose déjà délivrée.

Une telle pompe ne permet pas non plus de régler le débit d'une dose continue, mais uniquement d'administrer une dose instantanée. Les moyens magnétiques doivent être appliqués tout le temps de l'administration de la dose.

L'invention vise à pallier ces inconvénients.

Un but de l'invention est donc de fournir un dispositif d'injection de fluides permettant d'obtenir un débit de fluide variable dans le temps. Un autre but de l'invention est de fournir un dispositif comportant à la fois des éléments mécaniques et des éléments électroniques, les éléments mécaniques étant seuls implantés car bon marché, et les éléments électroniques étant externes, car chers, et donc récupérables du fait qu'ils ne sont pas implantés.

A cet effet, l'invention concerne un dispositif d'injection de fluides, apte à être implanté, du type comportant : un réservoir de fluide, une canalisation de sortie du fluide et des moyens de pompage du fluide dans le réservoir caractérisé en ce qu'il comporte en outre,

— une canalisation d'amenée du fluide,

— une canalisation intermédiaire disposée entre le réservoir et les canalisations d'amenée et de sortie,

— des moyens de commutation entre les canalisations d'amenée, de sortie et la canalisation intermédiaire,

— des moyens élastiques commandant le débit maximum désiré du fluide injecté,

— des moyens magnétiques agissant d'une part simultanément sur les moyens élastiques et sur les moyens de pompage et d'autre part sur les moyens de commutation.

Lorsque les moyens magnétiques sont actifs dans un sens, ils tendent les moyens élastiques et commandent les moyens de pompage qui délivrent une dose déterminée de fluide et sensiblement instantanée, lorsque les moyens magnétiques sont inactifs, les moyens de pompage sont alors commandés exclusivement par les moyens élastiques qui eux sont actifs. Les moyens de pompage délivrent une dose continue de fluide.

Enfin, lorsque les moyens magnétiques sont actifs dans le sens opposé, ils agissent sur les moyens de commutation qui notamment ouvrent plus ou moins la canalisation de sortie du fluide et qui par conséquent règlent le débit du fluide injecté.

On prévoit des moyens de débrayage des moyens élastiques avec les moyens magnétiques.

De préférence, les moyens de commutation sont constitués par une vanne trois voies qui a pour fonction d'ouvrir ou de fermer les canalisations.

Les moyens élastiques sont par exemple un ressort, tel qu'un ressort spiralé dont une extrémité est fixe et dont l'autre extrémité est mobile et commandée par les moyens magnétiques. Ceux-ci sont par exemple constitués par un barreau magnétique externe qui tourne sous l'action d'un champ magnétique externe.

Ainsi, en fonction de la force du ressort, on peut le tendre à l'aide des moyens magnétiques tournant dans un sens de manière plus ou moins importante en déplaçant son extrémité mobile. Puis le ressort étant débrayé des moyens magnétiques, il se détend et commande l'injection de fluide.

Enfin, des moyens de comptage sont prévus. Une première fonction des moyens de comptage est de matérialiser la force avec laquelle on déplace le point mobile du ressort. Une seconde fonction des moyens de comptage est d'indiquer la position des moyens de commutation et notamment l'ouverture et la fermeture des différentes canalisations.

On peut disposer un petit marteau qui tape sur une plaque métallique à chaque fois que les moyens magnétiques effectuent un certain nombre de tours.

Le marteau peut taper, par exemple une fois lorsque le barreau magnétique a effectué un tour dans un sens et peut taper par exemple deux fois lorsque le barreau magnétique effectue un tour en sens inverse.

Des moyens d'amplification sonores, tels qu'un micro, ont pour fonction d'amplifier le son émis par le choc du marteau sur la plaque et le son est ainsi perceptible de l'extérieur du dispositif.

Les moyens de pompage sont par exemple une pompe plongeante qui parvient dans le réservoir de fluide.

La description suivante, en regard des dessins annexés à titre d'exemples non limitatifs permettra de comprendre comment l'invention peut être mise en pratique.

La figure 1 est un schéma du dispositif d'injection de fluides selon l'invention.

La figure 2 est un graphique montrant d'une part la détente du ressort au cours du temps et d'autre part la dose injectée au cours du temps.

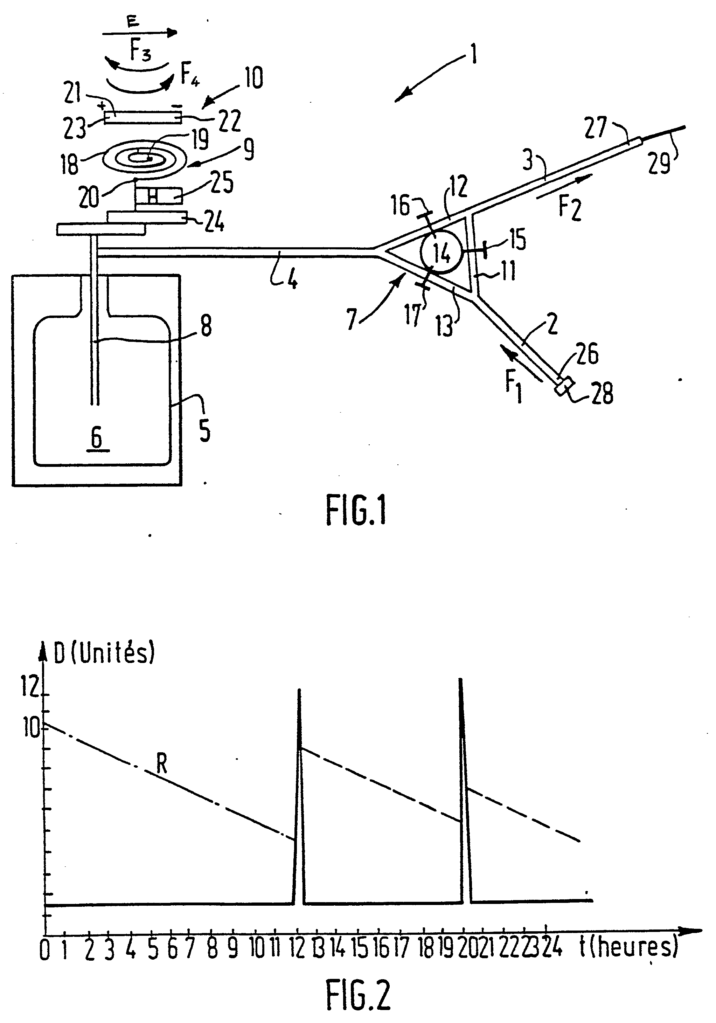

Le dispositif 1 d'injection de fluide représenté sur la figure 1 est destiné à être implanté par exemple à un patient atteint de diabète.

Le dispositif 1 comporte une canalisation d'amenée 2 de fluide et une canalisation de sortie 3 de fluide, une canalisation intermédiaire 4 est disposée entre un réservoir 5 de fluide 6 et les canalisations 2 et 3. Des moyens de commutation 7 ont pour fonction de mettre en communication ou non les canalisations 2, 3, 4. Des moyens de pompage 8 permettent de soutirer du fluide 6 pour l'envoyer dans la canalisation intermédiaire 4.

Le dispositif 1 comporte en outre des moyens élastiques 9 et des moyens magnétiques 10.

Les moyens magnétiques 10 agissent d'une part simultanément sur les moyens élastiques 9 et sur les moyens de pompage 8 et d'autre part sur les moyens de commutation 7.

Les moyens magnétiques 10 étant actifs dans un sens, ils tendent les moyens élastiques 9 et commandent les moyens de pompage 8 qui délivrent une dose déterminée de fluide, cette dose étant pratiquement instantanée. Les moyens élastiques 9 ainsi que les moyens de pompage 8 sont alors embrayés avec les moyens magnétiques 10 à l'aide de moyens d'embrayage/débrayage.

Lorsque les moyens magnétiques 10 sont inactifs, les moyens de pompage 8 sont commandés par les moyens élastiques 9 qui se détendent. Ces moyens élastiques 9 sont alors débrayés des moyens magnétiques 10. Les moyens de pompage 8 délivrent une dose continue de fluide qui est fonction de la force avec laquelle se détendent les moyens élastiques 9.

Lorsque les moyens magnétiques 10 sont actifs en sens opposé, ils agissent sur les moyens de commutation 7. Ainsi, les moyens de commutation 7 par exemple ouvrent partiellement les diverses canalisations. En fonction de l'ouverture et de la fermeture de la canalisation de sortie, on règle donc le débit du fluide injecté.

De préférence les canalisations d'amenée 2 et de sortie 3 sont réunies par une première conduite de liaison 11, les canalisations de sortie 3 et intermédiaire 4 sont réunies par une deuxième conduite de liaison 12, les canalisations intermédiaire 4 et d'amenée 2 par une troisième conduite de liaison 12, la canalisation intermédiaire 4 et d'amenée 2 par une troisième conduite de liaison 13. Chaque conduite de liaison 11, 12, 13 peut être ouverte ou fermée par un robinet central 14 tournant ou une vanne trois voies ou chacune par trois robinets 15, 16, 17.

Les moyens élastiques 9 sont, selon un mode de réalisation préféré de l'invention, constitués par un ressort 18 en spirale. Le ressort 18 comporte une extrémité fixe 19 et une extrémité mobile 20. L'extrémité mobile 20 est commandée par les moyens magnétiques 10.

Les moyens magnétiques 10 sont de préférence constitués par un barreau magnétique 21 muni de pôles négatifs 22 et positifs 23. Le barreau magnétique 21 tourne sous l'action d'un champ magnétique E externe, soit dans un sens, soit dans le sens opposé. Plus préférentiellement les moyens magnétiques 10 sont externes.

Les moyens élastiques 9 sont reliés aux moyens de pompage 8 par l'intermédiaire d'un système de pignons 24.

Enfin, des moyens de comptage 25 ont pour première fonction de matérialiser la force avec laquelle on tend les moyens élastiques et donc avec laquelle on déplace le point mobile 20 du ressort 18 par l'intermédiaire du champ magnétique E. A chaque fois que le barreau magnétique 21 effectue un nombre donné de tours dans un sens (flèche $F_3$, par exemple selon le sens de rotation des aiguilles d'une montre), les moyens de comptage 25, par exemple un petit marteau (non représenté) frappant une fois une plaque (non représentée) émettent un son qui est amplifié par des moyens amplificateurs, plus préférentiellement un micro.

Les moyens de comptage 25 ont pour seconde fonction de matérialiser le degré d'ouverture ou de fermeture des conduites de liaison 11, 12 et 13, l'ouverture et la fermeture étant effectuées lorsque le barreau magnétique 21 tourne dans le sens opposé (flèche $F_4$) au sens de rotation effectuée pour tendre le ressort 18. Ainsi le signal sonore indique la position du robinet trois voies. On prévoit par exemple le codage suivant :

| nombre de coups | conduite 11 | conduite 12 | conduite 13 |
|---|---|---|---|
| 2 | 0 | 0 | 0 |
| 3 | 0 | 1/4 | 0 |
| 4 | 0 | 1/2 | 0 |
| 5 | 0 | 3/4 | 0 |
| 6 | 0 | 1 | 0 |
| 7 | 1 | 0 | 0 |
| 8 | 0 | 0 | 1 |

1 = ouverture complète
0 = fermeture.

Lorsque le barreau magnétique 21 effectue une rotation en sens inverse (flèche $F_4$), le marteau tape au moins deux coups successifs sur la plaque.

Le son émis est perceptible de l'extérieur, ce qui permet donc de compter les coups. A un certain nombre de coups correspond un débit connu de fluide 6 injecté. Par exemple pour un nombre de coups égal à 6, le débit de fluide D est maximum, tandis que pour trois coups, il est de D/4, pour quatre coups il est de D/2 et pour cinq coups est de 3/4 D.

Les moyens de pompage 8 sont constitués par une pompe plongeante qui parvient dans le fluide 6.

Enfin, on prévoit aux extrémités libres respectives 26 et 27 des canalisations d'amenée 2 et de sortie 3 d'une part un embout 28 de raccordement avec une conduite externe et d'autre part une seringue ou un cathéter 29.

Le fonctionnement du dispositif 1 est le suivant :

Tous les moyens sont implantés dans le corps d'un patient. Le réservoir 6, la pompe 8, l'engrenage 24, le compteur 25, le ressort 18, les différentes canalisations 2, 3, 4 et conduites 12, 13, 14, la vanne trois voies 14 sont donc implantés. Seuls le barreau magnétique 21 et l'embout 28 de raccordement sont donc externes.

Le cathéter 29 parvient dans une veine par exemple ou dans le péritoine.

4

Dans l'exemple d'un patient atteint de diabète, le fluide injecté est de l'insuline. Le débit D d'insuline est fixé thérapeutiquement et est d'environ 1 unité par heure de façon continue et de 10 à 20 unités par heure au moment des repas.

Lorsque le dispositif 1 vient d'être implanté, on remplit le réservoir 5 en ouvrant le robinet 17 et fermant les robinets 15 et 16 et donc en tournant le barreau aimanté 21 dans le sens opposé au sens nécessaire pour tendre le ressort (sens inverse des aiguilles d'une montre). On compte le nombre de coups émis par le signal sonore et l'on sait que la conduite 13 est ouverte lorsque 8 coups sont émis.

Par l'embout 28 on introduit du fluide 6 qui parvient dans le réservoir 5 selon le sens de la flèche $F_1$.

On fait ensuite tourner le barreau magnétique 2 dans le sens des aiguilles d'une montre et tend le ressort 18 à une force donnée qui correspond à un débit de fluide donné, par exemple 1,2 unité par heure (voir figure 2). Le compteur 25 permet de connaître la force à laquelle on tend le ressort et donc le débit maximum D de fluide.

Si, lorsque l'on tend le ressort on ne désire pas injecter de fluide, on ferme auparavant toutes les conduites 11, 12, 13 et préalablement on compte 2 coups en tournant le barreau 21 en sens inverse du sens de rotation des aiguilles d'une montre.

Puis, le ressort 18 étant débrayé, le fluide est injecté selon le débit D. Si le débit souhaité est inférieur (1/3 D, 1/2 D, 3/4 D par ex) on agit auparavant en tournant le ressort selon le sens inverse et en comptant le nombre de coups émis, pour ouvrir plus ou moins la canalisation 12.

Lorsque le malade désire prendre un repas, par exemple au temps t = 12 h et t = 20 h, il agit sur le champ magnétique qui commande alors la pompe. Une dose instantanée de 13 unités par exemple est alors injectée tandis que le ressort 18 est remonté comme représenté par la courbe R de la figure 2.

Le dispositif selon l'invention permet un nettoyage facile du cathéter 29 et de la canalisation de sortie 3. Pour ce faire, on ferme les robinets 16 et 17 et on laisse le robinet 15 ouvert.

On introduit dans le sens de la flèche $F_1$ un liquide légèrement acide qui ressort selon la flèche $F_2$ par le cathéter 29. Les remontées provenant du péritoine et susceptibles de polluer le dispositif sont alors évacuées.

**Revendications**

1. Dispositif d'injection de fluide apte à être implanté, du type comportant un réservoir (5) de fluide, une canalisation de sortie (3) du fluide (6) et des moyens de pompage (8) du fluide dans le réservoir (5), caractérisé en ce qu'il comporte en outre en combinaison :
— une canalisation d'amenée (2) de fluide (6),
— une canalisation intermédiaire (4) disposée entre le réservoir (5) et les canalisations d'amenée (2) et de sortie (3),
— des moyens de commutation (14) entre les canalisations d'amenée (2), de sortie (3) et la canalisation intermédiaire (4),
— des moyens élastiques (9) commandant le débit maximum désiré (D) du fluide injecté,
— des moyens magnétiques (10) agissant d'une part sur les moyens de pompage (8) et d'autre part sur les moyens de commutation (14).

2. Dispositif selon la revendication 1, caractérisé par le fait que les moyens magnétiques (10), lorsqu'ils sont actifs dans un sens, tendent les moyens élastiques (9) et commandent les moyens de pompage (8) qui délivrent une dose déterminée de fluide sensiblement instantanée et lorsqu'ils sont actifs dans le sens opposé, agissent sur les moyens de commutation (7).

3. Dispositif selon l'une quelconque des revendications 1 et 2, caractérisé par le fait que lorsque les moyens magnétiques (10) sont inactifs, les moyens de pompage (8) sont commandés exclusivement par les moyens élastiques (9).

4. Dispositif selon la revendication 1, caractérisé par le fait que les moyens de commutation (14) sont constitués par une vanne trois voies (11, 12, 13).

5. Dispositif selon l'une quelconque des revendications 1 à 4, caractérisé par le fait que les moyens élastiques (9) sont constitués par un ressort (18), par exemple en spirale, dont une extrémité (19) est fixe et l'autre extrémité (20) est mobile et commandée par les moyens magnétiques (10).

6. Dispositif selon l'une quelconque des revendications 1 à 5, caractérisé par le fait que les moyens magnétiques (10) sont constitués par un barreau magnétique (21) ayant des pôles négatifs (22) et des pôles positifs (23) et tournant sous l'action d'un champ magnétique externe E.

7. Dispositif selon la revendication 5, caractérisé par le fait que les moyens magnétiques (10) sont externes.

8. Dispositif selon l'une quelconque des revendications 1 à 7, caractérisé par le fait qu'il comporte des moyens de comptage (25) dont la première fonction est de matérialiser la force selon laquelle on tend les moyens élastiques (9) et la seconde fonction est d'indiquer la position des moyens de commutation (14) et notamment l'ouverture de la fermeture des différentes canalisations (11, 12, 13).

9. Dispositif selon la revendication 8, caractérisé par le fait que les moyens de comptage (25) sont constitués par un marteau frappant sur une plaque, et des moyens d'amplification sonore.

10. Dispositif selon l'une quelconque des revendications 1 à 8, caractérisé par le fait qu'il comporte

5

un système d'engrenage à pignons (24) disposé entre les moyens élastiques (9) et les moyens de pompage (8).

11. Dispositif selon l'une quelconque des revendications 1 à 9, caractérisé par le fait que les moyens de pompage (8) sont constitués par une pompe plongeante.

## Claims

1. A device for injecting fluids, designed to be implanted, of the kind comprising a fluid reservoir (5), an outlet channel (3) for the fluid (6) and pumping means (8) for the fluid in the reservoir (5), characterised in that it also comprises in combination :
— an inlet channel (2) for the fluid (6),
— an intermediate channel (4) between the reservoir (5) and the inlet (2) and outlet (3) channels,
— switching means (14) between the inlet (2), outlet (3) and intermediate (4) channels,
— elastic means (9) controlling the maximum desired output (D) of the injected fluid,
— magnetic means (10) acting on the elastic means (9) and the pumping means (8) on the one part and the switching means (14) on the other part.

2. A device according to claim 1, characterised in that the magnetic means (10), when acting in one direction, stretch the elastic means (9) and control the pumping means (8) which release a determined dose of fluid substantially instantaneously and, when acting in the opposite direction, act on the switching means (7).

3. A device according to either one of claims 1 and 2, characterised in that when the magnetic means (10) are stationary, the pumping means (8) are controlled entirely by the elastic means (9).

4. A device according to claim 1, characterised in that the switching means (14) are comprised of a three-way valve (11, 12, 13).

5. A device according to any one of claims 1 to 4, characterised in that the elastic means (9) are comprised of a spring (18), for example a spiral spring, one end (19) of which is fixed and the other end (20) of which is movable and controlled by magnetic means (10).

6. A device according to any one of claims 1 to 5, characterised in that the magnetic means (10) are comprised of a bar magnet (21) having negative poles (22) and positive poles (23) and turning under the action of an external magnetic field E.

7. A device according to claim 5, characterised in that the magnetic means (10) are external.

8. A device according to any one of claims 1 to 7, characterised in that it comprises counting means (25), the primary function of which is to create the force to stretch the elastic means (9) and the secondary function is to indicate the position of the switching means (14) and in particular the beginning of closure of the different channels (11, 12, 13).

9. A device according to claim 8, characterised in that the counting means (25) are comprised in a hammer hitting a plate, and sound amplifying means.

10. A device according to any one of claims 1 to 8, characterised in that it comprises a pinion gear system (24) disposed between the elastic means (9) and the pumping means (8).

11. A device according to any one of claims 1 to 9, characterised in that the pumping means (8) are comprised of a submergeable pump.

## Patentansprüche

1. Implantierbare Vorrichtung zur Injektion von Flüssigkeiten, mit einem Flüssigkeitsbehälter (5), mit einer Ausgangsleitung (3) für die Flüssigkeit (6) und mit einem Flüssigkeits-Pumpenmittel (8) im Behälter (5), dadurch gekennzeichnet, daß sie ferner in Kombination folgendes aufweist :
Eine Zulaufleitung (2) für die Flüssigkeit (6),
eine Zwischenleitung (4) zwischen dem Behälter (5) und der Zulaufleitung (2) und der Ausgangsleitung (3)
ein Umschaltmittel (14) zwischen der Zulaufleitung (2), der Ausgangsleitung (3) und der Zwischenleitung (4),
ein Ferdermittel (9), das die maximal gewünschte Menge (D) an injizierter Flüssigkeit steuert, und
ein Magnetmittel (10), das einerseits auf das Federmittel (9) und auf das Pumpenmittel (8) und andererseits auf das Umschaltmittel (14) wirkt.

2. Vorrichtung nach Anspruch 1, dadurch gekennzeichnet, daß das Magnetmittel (10), sobald es in einer Richtung wirksam ist, das Federmittel (9) spannen und das Pumpenmittel (8) bestätigt, das eine bestimmte Flüssigkeitsmenge im wesentlichen sofort abgibt, und, sobald es in der entgegengesetzten Richtung wirksam ist, auf das Umschaltmittel (7) einwirkt.

3. Vorrichtung nach Anspruch 1 oder 2, dadurch gekennzeichnet, daß sobald das Magnetmittel (10) inaktiv ist, das Pumpenmittel (8) ausschließlich durch das Federmittel (9) betätigt ist.

4. Vorrichtung nach Anspruch 1, dadurch gekennzeichnet, daß das Umschaltmittel (14) durch ein Dreiwege-Ventil (11, 12, 13) gebildet sind.

5. Vorrichtung nach einem der Ansprüche 1 bis 4, dadurch gekennzeichnet, daß das Federmittel (9) durch eine Feder (18), bspw. eine Spiralfeder gebildet ist, deren eines Ende (19) ortsfest ist und deren anderes Ende (20) beweglich und durch das Magnetmittel (10) betätigbar ist.

6. Vorrichtung nach einem der Ansprüche 1 bis 5, dadurch gekennzeichnet, daß das Magnetmittel (10) durch einen Magnetstab (21) gebildet ist, der negative Pole (22) und positive Pole (23) aufweist und der sich unter der Wirkung eines äußeren Magnetfeldes (E) dreht.

7. Vorrichtung nach Anspruch 5, dadurch gekennzeichnet, daß das Magnetmittel (10) estern ist.

8. Vorrichtung nach einem der Ansprüche 1 bis 7, dadurch gekennzeichnet, daß sie ein Zählmittel (25) aufweist, dessen erste Funktion darin besteht, die Kraft anzugeben, mit welcher das Federmittel (9) gespannt ist, und dessen zweite Funktion darin besteht, die Position des Umschaltmittels (14) und insbesondere den Öffnungsgrad der Verschlußvorrichtung der verschiedenen Leitungen (11, 12, 13) anzuzeigen.

9. Vorrichtung nach Anspruch 8, dadurch gekennzeichnet, daß das Zählmittel (25) durch ein Hammerwerk, das auf eine Platte schlägt, und durch Klangverstärkungsmittel gebildet ist.

10. Vorrichtung nach einem der Ansprüche 1 bis 8, dadurch gekennzeichnet, daß sie ein Zahnradgetriebe (24) aufweist, das zwischen dem Federmittel (9) und dem Pumpenmittel (8) angeordnet ist.

11. Vorrichtung nach einem der Ansprüche 1 bis 9, dadurch gekennzeichnet, daß das Pumpenmittel (8) durch eine Tauchpumpe gebildet ist.

FIG.1

FIG.2